## Europäisches Patentamt

## European Patent Office

## Office européen des brevets

(19)

(11) Veröffentlichungsnummer : **0 323 527 B1**

(12) **EUROPÄISCHE PATENTSCHRIFT**

(45) Veröffentlichungstag der Patentschrift :
**04.03.92 Patentblatt 92/10**

(51) Int. Cl.⁵ : **A61M 1/00**

(21) Anmeldenummer : **88906215.4**

(22) Anmeldetag : **17.03.88**

(86) Internationale Anmeldenummer :
**PCT/SU88/00059**

(87) Internationale Veröffentlichungsnummer :
**WO 89/00054 12.01.89 Gazette 89/02**

(54) **EINRICHTUNG ZUM SAMMELN EINER ABGESAUGTEN SUBSTANZ.**

(30) Priorität : **02.07.87 SU 4274863**

(43) Veröffentlichungstag der Anmeldung :
**12.07.89 Patentblatt 89/28**

(45) Bekanntmachung des Hinweises auf die
Patenterteilung :
**04.03.92 Patentblatt 92/10**

(84) Benannte Vertragsstaaten :
**CH DE FR GB IT LI**

(56) Entgegenhaltungen :
**DE-B- 1 947 123**
**SU-A- 544 435**
**US-A- 451 673**
**US-A- 3 773 211**
**US-A- 3 889 657**

(73) Patentinhaber :
**NAUCHNO-PROIZVODSTVENNOE**
**OBIEDINENIE 'MEDINSTRUMENT'**
**ul. K.Tinchurina, 31**
**Kazan, 420022 (SU)**

(72) Erfinder : **GAINUTDINOVA, Raisa**
**Vladimirovna**
**ul. Spartakovskaya, 80-15**
**Kazan, 420049 (SU)**
Erfinder : **ZHUKOVSKY, Yakov Grigorievich**
**ul. Krupskoi, 14-76**
**Moscow, 117311 (SU)**
Erfinder : **PETROVA, Vera, Mitrofanovna**
**ul. Frunze, 13a-2**
**Kazan, 420023 (SU)**
Erfinder : **KHUSAINOV, Nail, Tagirovich**
**ul. Ju. Fuchika, 42-129**
**Kazan, 420110 (SU)**

(74) Vertreter : **Nix, Frank Arnold, Dr.**
**Kröckelbergstrasse 15**
**W-6200 Wiesbaden (DE)**

EP 0 323 527 B1

**Beschreibung**

Technisches Gebiet

Die vorliegende Erfindung bezieht sich auf Mittel zur Schwangerschaftsunterbrechung im frühen Stadium und auf die Endometriumbiopsie, insbesondere auf eine Einrichtung zum Sammeln der abgesaugten Substanz.

Die Erfindung gestattet es, Informationen über die Menge und Qualität der abgesaugten Substanz zu erhalten.

Zugrundeliegender Stand der Technik

Bekannt ist eine Einrichtung zum Sammeln der abgesaugten Substanz (US, A, Nr. 4334538), die ein Gehäuse einschließt, das entlang seiner Längsachse gestreckt ist, einen zentralen Innenraum mit einem in dessen Innerem befindlichen Kanal besitzt. Am Gehäuseboden ist ein Behälter zum Sammeln der abgesaugten Substanz befestigt, der mit dem zentralen Gehäuseinnenraum in Verbindung steht und senkrecht zur Längsachse des Gehäuses angeordnet ist. Der Kanal erstreckt sich über die gesamte Gehäuselänge und verbindet den zentralen Innenraum mit den entgegengesetzten Gehäuseenden.

In der Gehäusewand ist eine Durchgangsbohrung vorhanden, die den Druck regelt und den zentralen Gehäuseinnenraum mit der Atmosphäre verbindet. Das eine Kanalende steht mit einer Vakuumquelle, das andere dagegen mit der Absaugtülle in Verbindung.

Die bekannte Einrichtung kennzeichnet sich dadurch, daß nach dem Anschließen an die Vakuumquelle und die in die Gebärmutterhöhle eingeführte Absaugtülle der Arzt mittels der letzteren außer hin- und hergehenden Bewegungen auch eine Umdrehung um die Längsachse bewerkstelligt. Dabei kippt der Behälter um, die abgesaugte Substanz fließt aus und gelang in das Vakuumsystem, was im weiteren die quantitative und qualitative Analyse der abgesaugten Substanz erschwert.

Die bekannte Einrichtung ist auch dadurch gekennzeichnet, daß das Gehäuse eine von der zylindrischen verschiedene Form besitzt, was die Umdrehung stört. Die Umdrehung des Gehäuses ist dabei ungleichmäßig, wodurch sich Teile des befruchteten Eies in der Gebärmutterhöhle halten können.

Alles das hat eine Verlängerung der Operationsdauer zur Folge.

Es ist eine Einrichtung zum Sammeln der abgesaugten Substanz bekannt (DE, B, 1947123), die ein Gefäß mit Deckel darstellt. Im Gefäßdeckel ist eine Durchgangsbohrung vorgesehen, die mit dem Gefäßinnenraum und dem distalen Ende der Absaugtülle über ein Verbindungselement in Verbindung gesetzt ist. Die Achse der Durchgangsbohrung ist relativ zur Gefäßachse versetzt. Das Gefäß besteht aus einem polymeren Material. Im Gefäß ist ein zylindrisches Netzelement konzentrisch angeordnet, das ein Rohr mit radialen Durchgangsbohrungen umfaßt. Der Rohrinnenraum steht mit einer Vakuumquelle in Verbindung.

Die in die Sammeleinrichtung kommende abgesaugte Substanz verliert ihren flüssigen Bestandteil, der durch das zylindrische Netzelement in das Vakuumsystem hindurchsickert.

Die bekannte Einrichtung ist dadurch gekennzeichnet, daß der Verlust des flüssigen Bestandteils der abgesaugten Substanz die quantitative Analyse der anfallenden Substanz erschwert.

Die bekannte Einrichtung kennzeichnet sich ferner dadurch, daß die exzentrische Lage der Absaugtülle keine gleichmäßige Umdrehung des Gehäuses mit der Absaugtülle zu erzielen erlaubt, was zum Zurückbleiben von Teilen des befruchteten Eies in der Gebärmutterhöhle führt. All dies bedingt eine Verlängerung der für die Operation benötigten Zeit.

Die bekannte Einrichtung ist auch noch dadurch gekennzeichnet, daß das zylindrische Netzelement nach der Behandlung der Operation nur schwer von haftengebliebenen dichten Teilchen der abgesaugten Substanz gereinigt werden kann. Bei der Reinigung werden diese Teilchen verletzt, was die weitere histologische Untersuchung derselben erschwert.

Weitere Beispiele von Einrichtung zum Sammeln von abgesaugten Substanzen sind in US-A-3 889 657 und in US-A-3 773 211 beschrieben.

Offenbarung der Erfindung

Der Erfindung liegt die Aufgabe zugrunde, eine Einrichtung zum Sammeln der abgesaugten Substanz mit einer solchen Anordnung des Gefäßes im Gehäuse und mit einer solchen Verbindung der Absaugtülle mit dem Gehäusedeckel zuschaffen, die es erlauben würde, die bei der Operation abgesaugte Substanz voll aufzunehmen, eine individuelle quantitative und qualitative Analyse derselben durchzuführen sowie die für die postoperative Behandlung des Gefäßes erforderliche Zeit zur Gewährleistung eines kontinuierlichen Betriebs zu verkürzen.

Die gestallte Aufgabe wird dadurch gelöst, daß in der Einrichtung zum Sammeln der abgesaugten Substanz, die ein Gefäß mit Deckel enthält, dessen Innenraum mit einer Vakuumquelle in Verbindung steht und über eine im Deckel vorgesehene Durchgangsbohrung mit einer Absaugtülle verbunden ist, erfindungsgemäß das Gefäß mit Deckel in einem Gehäuse mit Deckel konzentrisch angeordnet ist, derart, daß zwischen der Gehäuseinnenwand und der Gefäßaußenwand ein mit der Vakuumquelle in Verbindung stehender Ringspalt gebildet, die Durchgangsbohrung im Gefäßdeckel gleichachsig zum Ringspalt angebracht und der Gehäusedeckel mit einem Stutzen versehen ist, bei dem das eine Ende zum Verbindung mit dem proximalen Ende der Absaugtülle bestimmt ist, während das andere Ende ins Innere des Gehäuses zeigt, gleichachsig zur im Zylinderdeckel vorhandenen Bohrung angeordnet und vom Gefäß umfaßt ist.

Diese Ausführung der Einrichtung zum Sammeln der abgesaugten Substanz gestattet es, die gesamte bei der Operation abgesaugte Substanz verlustlos zu sammeln. Dies ermöglicht eine rasche quantitative und qualitative Analyse derselben.

Eine derartige Ausführung läßt kein Eindringen der abgesaugten Substanz in die Verbindungselemente und Vakuumsystemelemente zu, was bei der AIDS-Prophylaxe von großer Bedeutung ist, weil dadurch die Ausbreitung der Infektion verhindert wird, die sich in Form von Belägen, Spritzern, Klumpen von geronnenem Blut auf den Verbindungselementen absetzt, die für die postoperative Behandlung schwer zugänglich sind.

Dabei läßt sich das Gefäß in der erfindungsgemäß vorgeschlagenen Einrichtung aus dem Gehäuse unschwer entnehmen, mühelos und rasch desinfizieren, wonach es erneut bei gesicherter Unterbindung der Infektionsausbreitung zur Anwendung bereit ist. Dies verkürzt die Dauer der postoperativen Behandlung des Gefäßes zur Gewährleistung eines kontinuierlichen Einsatzes.

Zweckmäßigerweise soll ferner der Durchgangsquerschnitt des zur Verbindung mit dem proximalen Ende der Absaugtülle bestimmten Stutzenendes größer als der Durchgangsquerschnitt des ins Innere des Gehäuses zeigenden Stutzenendes sein.

Diese Ausführung des Stutzens trägt zur guten Fixierung der Absaugtülle bei und läßt kein Verspritzen der abgesaugten Substanz über die Innenwände des Gefäßes zu, wodurch die bei der Operation abgesaugte Substanz verlustlos gesammelt werden kann.

## Kurze Beschreibung der Zeichnungen

Im folgenden wird die Erfindung durch ein konkretes Beispiel ihrer Ausführung unter Bezugnahme auf beiliegenden Zeichnungen erläutert; in diesen zeigt:

Fig. 1 eine Einrichtung zum Sammeln der abgesaugten Substanz, gemäß der Erfindung, im Längsschnitt;
Fig. 2 eine Einrichtung zum Sammeln der abgesaugten Substanz in demontierten Zustand, im Längsschnitt;
Fig. 3 eine Ausführungsform eines Teiles des Gefäßes mit einem in Form eines abgestumpften Kegels ausgebildeten Deckel, wobei die kegelförmige Deckelwand ins Innere des Gefäßes zeigt, gemäß der Erfindung, im Längsschnitt.

## Beste Ausführungsform der Erfindung

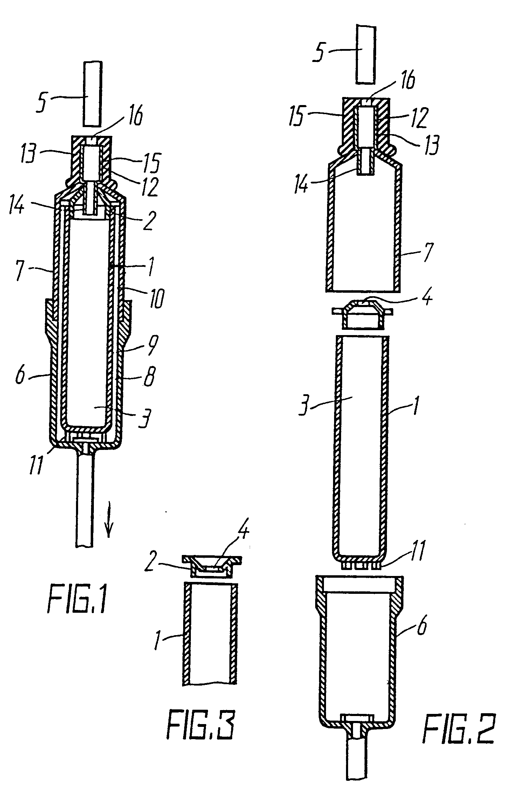

Die Einrichtung zum Sammeln der abgesaugten Substanz enthält ein Gefäß 1 (Fig. 1) mit Deckel 2. Der Innenraum 3 des Gefässes 1 steht mit einer (in der Zeichnung nicht gezeigten) Vakuumquelle in Verbindung. Im Deckel 2 ist eine Durchgangsbohrung 4 (Fig. 2) vorhanden, durch die der Innenraum 3 (Fig. 1) des Gefäßes 1 mit einer Absaugtülle 5 verbunden ist. Der Deckel 2 des Gefäßes 1 ist als abgestumpfter Kegel ausgebildet und kann mit seinem kegeligen Teil nach außen (Fig. 2) bzw. ins Innere des Innenraums 3 des Gefäßes 1 (Fig. 3) zeigen. Das Gefäß 1 kann aus einem transparenten polymeren Material ausgeführt sein, was dem Arzt die Möglichkeit gibt, den Füllungsgrad desselben mit der abgesaugten Substanz zu kontrollieren.

Das Gefäß 1 (Fig. 1) mit dem Deckel 2 ist in einem Gehäuse 6 mit einem Deckel 7 konzentrisch angeordnet, derart, daß zwischen der Innenwand 8 des Gehäuses 6 und der Außenwand 9 des Gefäßes 1 ein Ringspalt 10 gebildet ist, der mit einer Vakuumquelle in Verbindung steht. Die Durchgangsbohrung 4 im Deckel 2 des Gefäßes 1 ist gleichachsig zum Ringspalt 10 ausgeführt.

An der Außenfläche des Bodens des Gefäßes 1 sind Stützelemente 11 (Fig. 2) vorhanden, die zum bessern Luftdurchtritt beitragen.

Der Deckel 7 des Gehäuses 6 ist mit einem Stutzen 12 versehen, der jeweils ein äußeres Ende 13 und ein inneres Ende 14 besitzt. Dabei ist der Durchgangsquerschnitt des inneren Endes 14 des Stutzens 12 kleiner als der Durchgangsquerschnitt des äußeren Endes 13 desselben. Das äußere Ende 13 des Stutzens 12 ist zur Fixierung der Ab saugtülle 5 bestimmt. Auf das äussere Ende 13 des Stutzens 12 wird eine Gummihaube 15 mit einer Öffnung 16 aufgesetzt, deren Durchmesser kleiner als der Außendurchmesser der Absaugtülle 5 ist. Der verkleinerte Durchgangsquerschnitt des inneren Endes 14 des Stutzens 12 gewährleistet eine sichere Fixierung der Absaugtüllen unterschiedlichen Durchmessers.

Das innere Ende 14 des Stutzens 12 liegt gleichachsig zur Bohrung 4 im Deckel 2 des Gefäßes 1 und ragt durch die Bohrung 4 frei in den Innenraum 3 des Gefäßes 1 hinein.

Die Einrichtung zum Sammeln der abgesaugten Substanz arbeitet auf die folgende Weise.

Das Gefäß 1 mit dem geöffneten Deckel 2 wird konzentrisch im Gehäuse 6 mit dem Boden zur Stirnseite desselben angeordnet, die mit einer Vakuumquelle verbunden ist. Das Gefäß 1 und das Gehäuse 6 werden mit den Deckeln 2 und 7 verschlossen. Dabei ragt das innere Ende 14 Stutzens 12 durch die Bohrung 4 des Deckels 2 in den Innenraum 3 des Gefäßes 1 hinein. Zur besseren Fixierung der Absaugtülle 5 sowie zur Gewährleistung der hermetischen Abdichtung der Einrichtung wird auf das äußere Ende 13 des Stutzens 12 eine Gummihaube 15 aufgesetzt, durch deren Öffnung 16 die in die Gebärmutterhöhle einzuführende Absaugtülle 5 in das äußere Ende 13 des Stutzens 12 bis zum Anschlag eingesteckt wird.

Der Arzt nimmt das Gehäuse 6 mit der Hand, schaltet das Vakuum ein und erteilt dem Gehäuse 6 mit der Absaugtülle 5 hin- und hergehende, schwingende und kreisende Bewegungen um die Längsachse des Gehäuses 6. Bei der Rückbewegung der Absaugtülle 5, wenn aus dem äußeren Muttermund ein kleiner Teil der Nut der Absaugtülle 5 zum Vorschein kommt, gleicht sich der Druck aus, und die abgesaugte Substanz gelangt mit einem Ruck in das Gefäß 1. Damit die Spritzer derselben auf die Innenwände 8 des Gehäuses 6 nicht gelangen, ist der Quer schnitt des inneren Endes 14 des Stutzens 12 gegenüber dem des äußeren Endes 13 kleiner gemacht.

Nach der Beendigung der Operation wird vom äußeren Ende 13 des Stutzens 12 die Absaugtülle 5 abgenommen, der Deckel 7 des Gehäuses 6 wird geöffnet, das Gefäß 1 mit dem Deckel 2 entnommen und durch einen neuen ersetzt, worauf eine nächste ähnliche Operation eingeleitet werden kann.

Die abgesaugte Substanz im eben entnommenen Gefäß wird einer quantitativen und qualitativen Analyse unterzogen, das Gefäß ausgespült und in eine Desinfektionslösung getaucht, wonach es bei den nachfolgenden Operationen erneut eingesetzt werden kann.

Auf diese Weise ist dank der im vorstehenden beschriebenen Ausführung der Einrichtung zum Sammeln der abgesaugten Substanz ein Verlust der abgesaugten Substanz aus dem Gefäß ausgeschlossen, es läßt sich eine vollständige individuelle quantitative und qualitative Analyse derselben rasch durchführen und kann eine ununterbrochene Einrichtungsfunktion dank der Verkürzung der für die post-operative Behandlung des Gefäßes benötigten Zeit gewährleistet werden.

Gewerbliche Verwertbarkeit

Am effektivsten kann die vorliegende Erfindung bei der Schwangerschaftsunterbrechung bei einer Zeitdauer bis zu 20 Tagen angewandt werden. Beim Ausfüllen des Gefäßes mit der abgesaugten Substanz wird auf seine Auswechselung höchstens 10 bis 15 s verwendet. Somit kann der Arzt 40 und mehr Operationen während einer Arbeitsschicht durchführen.

**Patentansprüche**

1. Einrichtung zum Sammeln einer abgesaugten Substanz, die ein Gefäß (1) mit Deckel (2) enthält, dessen Innenraum (3) mit einer Vakuumquelle in Verbindung steht und über eine im Deckel (2) vorgesehene Durchgangsbohrung (4) mit einer Absaugtülle (5) verbunden ist, dadurch **gekennzeichnet**, daß das Gefäß (1) mit Deckel (2) in einem Gehäuse (6) mit Deckel (7) konzentrisch angeordnet ist, derart, daß zwischen der Innenwand (8) des Gehäuses (6) und der Außenwand (9) des Gefäßes (1) ein mit der Vakuumquelle verbundener Ringspalt (10) gebildet ist, die Durchgangsbohrung (4) im Deckel (2) des Gefäßes (1) gleichachsig zum Ringspalt (10) liegt und der Deckel (7) des Gehäuses (6) mit einem Stutzen (12) versehen ist, bei dem das eine Ende (13) zur Verbindung mit dem proximalen Ende der Absaugtülle (5) bestimmt ist, während das andere Ende (14) des Stutzens (12) ins Innere des Gehäuses (6) zeigt, gleichachsig zur Bohrung (4) im Deckel (2) des Gefäßes (1) angeordnet und von diesem letzteren umfaßt ist.

2. Einrichtung zum Sammeln der abgesaugten Substanz nach Anspruch 1, dadurch **gekennzeichnet**, daß der Durchgangsquerschnitt des zur Verbindung mit dem proximalen Ende der Absaugtülle (5) bestimmten Endes (13) des Stutzens (12) größer als der Durchgangsquerschnitt des ins Innere des Gehäuses (6) zeigenden Endes (14) des Stutzens (12) ist.

## Claims

1. Device for collecting an aspirated substance, which contains a vessel (1) with lid (2), whose interior (3) is connected to a vacuum source and is connected via a drilled passage (4) provided in the lid (2) to an aspiration nozzle (5), characterised in that the vessel (1) with lid (2) is concentrically arranged in a housing (6) with lid (7) in such a manner that an annular gap (10) which is connected to the vacuum source is formed between the inner wall (8) of the housing (6) and the outer wall (9) of the vessel (1), the drilled passage (4) in the lid (2) of the vessel (1) is in the same axis as the annular gap (10), and the lid (7) of the housing (6) is provided with a connector (12) in which one end (13) is intended for connection to the proximal end of the aspiration nozzle (5), while the other end (14) of the connector (12) points into the interior of the housing (6), is arranged in the same axis as the drilling (4) in the lid (2) of the vessel (1) and is enveloped by this latter.

2. Device for collecting the aspirated substance according to Claim 1, characterised in that the cross-section of the passage in the end (13), which is intended for connection to the proximal end of the aspiration nozzle (5), of the connector (12) is greater than the cross-section of the passage in the end (14), which points into the interior of the housing (6), of the connector (12).

## Revendications

1. Dispositif pour ramasser des substances aspirées, comprenant un cylindre (1), doté d'un couvercle (2), dont la cavité (3) est en communication avec une source de vide et reliée à un embout d'aspiration (5) par l'intermédiaire d'un orificedébouchant (4) prévu dans le couvercle (2), caractérisé en ce que le cylindre (1) doté du couvercle (2) est monté de façon coaxiale dans un corps (6) doté d'un couvercle (7) de façon qu'entre la paroi intérieure (8) du corps (6) et la paroi extérieure (9) du cylindre (1) se forme un jeu annulaire (10) communiquant avec la source de vide, en ce que l'orifice débouchant (4) prévu dans le couvercle (2) du cylindre (1) est coaxial au jeu annulaire (10) et que le couvercle (7) du corps (6) est doté d'une tubulure (12) dont l'une des extrémité (13) est destinée à être reliée à l'extrémité rapprochée de l'embout d'aspiration (5), tandis que l'autre extrémité (14) de la tubulure (12), orientée à l'intérieure du corps (6), est coaxiale à l'orifice (4) prévu dans le couvercle (2) du cylindre (1) et est entourée par ce dernier.

2. Dispositif pour ramasser des substances aspirées selon la revendication 1, caractérisée en ce que la section de passage de l'extrémité (13) de la tubulure (12) destinée à être reliée à l'extrémité rapprochée de l'embout d'aspiration (5) est plus grande que la section de passage de l'extrémité (14) de la tubulure (12) orientée à l'intérieur du corps (6).

FIG.1

FIG.3

FIG.2